# EUROPEAN PATENT SPECIFICATION

(11) **EP 2 742 896 B1**
(45) Date of publication and mention of the grant of the patent: **08.08.2018**
(21) Application number: 12821622.3
(22) Date of filing: 13.08.2012
(51) Int. Cl.: A61C 3/02, A61B 17/16

(54) **AUTOGENOUS BONE COLLECTOR FOR HARVESTING AUTOGENOUS BONE**
SAMMELGERÄT FÜR AUTOGENE KNOCHENENTNAHME
DISPOSITIF COLLECTEUR D'AUTOGREFFE POUR PRÉLÈVEMENT D'OS AUTOGÈNE

(30) Priority: 11.08.2011 KR 20110080311; 24.08.2011 KR 20110084813
(43) Date of publication of application: 18.06.2014
(73) Proprietor: Osstemimplant Co., Ltd., Seoul 153-759 (KR)
(72) Inventor: MOON, Jong Hoon, Busan 611-749 (KR); LEE, Tae Euk, Busan 614-754 (KR); EOM, Tae Gwan, Busan 612-030 (KR)
(74) Representative: Carangelo, Pierluigi
(86) International application number: PCT/KR2012/006427
(87) International publication number: WO 2013/022324

(56) References cited:
- EP-A2- 2 138 255
- KR-A- 20110 016 602
- KR-A- 20110 016 602
- KR-B1- 100 813 434
- KR-B1- 100 813 434
- KR-B1- 100 838 942
- KR-B1- 101 013 285
- US-A- 4 830 000
- US-A1- 2004 210 229
- US-A1- 2010 196 844

## Description

### TECHNICAL FIELD

The present invention relates to an autogenous bone collector for collecting bone particles during implant surgery, the autogenous bone collector having a higher capacity of housing bone particles, and being capable of easily separating collected bone particles from a cutting unit, controlling a depth of the cutting unit that penetrates into an autogenous bone, and housing bone particles collected by the drill within the drill cover while preventing these from falling out of the drill cover.

### BACKGROUND ART

During dental implant surgery, bone particles are collected from a patient and the collected bone particles are then implanted into the patient. The bone particles are collected from an autogenous bone of the patient by using a drill attached to a dental handpiece. Korean Utility Model Registration No. 20-0406694 registered on January 13, 2006 discloses an autogenous bone collecting device.

However, when the autogenous bone collecting device is used, it is inconvenient to remove bone particles stuck in threads of a drill during collection of bone particles of a patient.

### DETAILED DESCRIPTION OF THE INVENTION

### TECHNICAL PROBLEM

The present invention provides an autogenous bone collector for having an increased capacity of housing bone particles, and being capable of easily separating bone particles from a cutting unit, controlling a depth of the cutting unit penetrating into an autogenous bone, and housing bone particles collected by the drill within the drill cover while preventing these from falling out of the drill cover.

### ADVANTAGEOUS EFFECTS

According to example embodiments of the present invention, an autogenous bone collector for collecting bone particles during implant surgery, includes a drill and a drill cover, has increased capacity of housing bone particles, and is capable of easily separating bone particles from a cutting unit, controlling a depth of the cutting unit that penetrates into an autogenous bone, and housing bone particles collected by the drill within the drill cover while preventing these from falling out of the drill cover.

### DESCRIPTION OF THE DRAWINGS

The above and other features and advantages of the present invention will become more apparent by describing in detail example embodiments thereof with reference to the attached drawings in which:
FIG. 1 is a front view of a drill of an autogenous bone collectoraccording to an embodiment of the present invention;
FIG. 2 is a bottom view of the drill shown in FIG. 1;
FIG. 3 is a sectional view taken along a line IV-IV of FIG. 1;
FIG. 4 is a front view of an autogenous bone collector according to an embodiment of the present invention;
FIG. 5 is a front view of a drill cover of the autogenous bone collector shown in FIG. 4;
FIG. 6 is a plan view of the drill cover shown in FIG. 5;
FIG. 7 is a sectional view of the drill cover shown in FIG. 5;
FIG. 8 is a diagram showing in closer detail a section slightly different from section "A" of FIG. 7;
FIG. 9 is a diagram showing a primary combination of the drill and the drill cover of the autogenous bone collector according to an embodiment of the present invention;
FIG. 10 is a sectional view taken along a line C-C of FIG. 4;
FIG. 11 is a front view of a drill according to another embodiment of the present invention;
FIG. 12 is a photograph of bone particles collected by using an existing autogenous bone collector; and
FIG. 13 is a photograph of bone particles collected by using an autogenous bone collector according to an embodiment of the present invention.

### BEST MODE

According to an aspect of the present invention, there is provided an autogenous bone collector including a drill having a shaft unit connected to a driving device, and a cutting unit, which rotates and collects bone particles of a patient; and a drill cover, which includes a main body that has the top and the bottom open and is coupled to the exterior of the cutting unit, wherein the main body is compressible in a direction in which the upper portion and the lower portion of the main body approach to-each other.

A cut-open portion, which includes spiral cuts, may be formed in the main body. The width of the cut-open portion may be constant.

The cut-open portion may include a first cut-open portion having a constant first cut-open width; and a second cut-open portion having a second cut-open width smaller than the first cut-open width.

The second cut-open portion may extend from the two opposite ends of the first cut-open portion, and the width of the second cut-open portion may gradually decrease toward an end portion thereof.

The autogenous bone collector may further include protruding parts protruding upward from the upper end of the drill cover spaced apart from each other at a constant interval in the circumferential direction.

The autogenous bone collector may further include a partitioning wall, which extends from the inner circumferential surface of the drill cover, wherein the partitioning wall may include a housing portion through which the cutting surface of the cutting unit pass.

Hook protrusions that are bent inward are arranged at the upper end of main body.

The main body may be formed of titanium nitride or stainless steel.

The cross-sectional area of the cutting unit may be from 10% to 40% of an area of an imaginary circle having a radius corresponding to a distance from the center of the cutting unit to the outermost end portion of the cutting unit.

The cutting unit may include a stopper protruding from the outer circumferential surface of the drill, movement of a drill cover may be restricted by the stopper, and a first protrusion may be formed below the stopper.

A second protrusion may be formed below the first protrusion.

The cutting unit may include a first blade, which spirally extends; and a second blade, which spirally extends at a same pitch as the first blade.

The end portion of the cutting unit may include two or more discontinuously sloped surfaces having different slopes.

The cutting unit may include a point that protrudes from the center portion of the end portion of the cutting unit.

### MODE OF THE INVENTION

The present invention relates to an autogenous bone collector for collecting bone particles during dental implant surgery.

Hereinafter, example embodiments of the present invention will be described in detail with reference to the attached drawings in which;

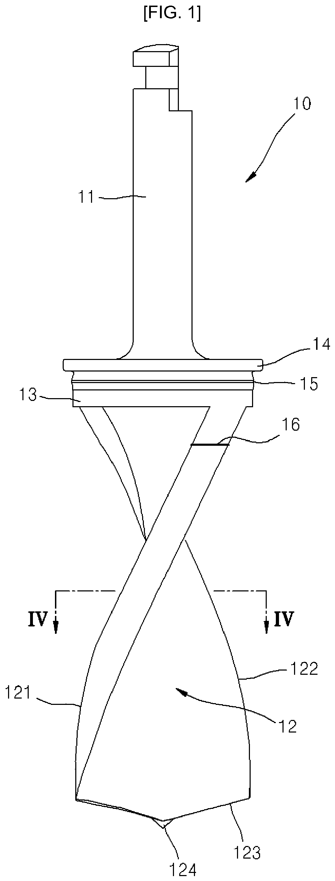

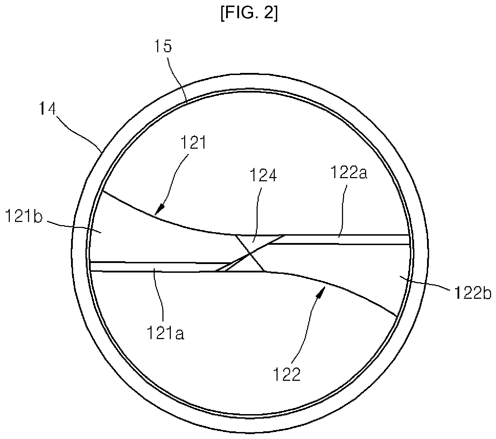

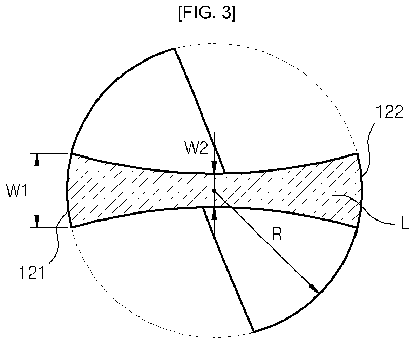

FIG. 1 is a front view of a drill of the autogenous bone collectoraccording to an embodiment. FIG. 2 is a bottom view of the drill shown in FIG. 1. FIG. 3 is a sectional view taken along a line IV-IV of FIG. 1.

Referring to FIG. 1, a drill 10 includes a shaft unit 11 and a cutting unit 12. The shaft unit 11 is connected to a driving device and the cutting unit 12 rotatably collects bone particles from a patient. A cross-sectional area L of the cutting unit 12 is from 10% to 40% of an area of an imaginary circle having a radius R corresponding to a distance from a center of the cutting unit 12 to an outermost end portion of the cutting unit 12.

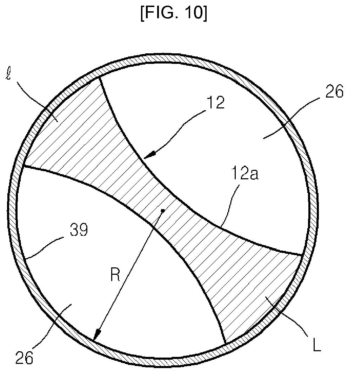

If the cross-sectional area L of the cutting unit 12 is less than 10% of the area of the imaginary circle, a strength of the cutting unit 12 decreases. If the cross-sectional area L of the cutting unit 12 is greater than 40% of the area of the imaginary circle, a storage space 26 (see FIG. 10) for housing collected bone particles becomes smaller due to an increase of a space occupied by the cutting unit 12.

The cross-sectional area L of the cutting unit 12 according to the present embodiment is 20% of the imaginary circle having the radius R corresponding to the distance from the center of the cutting part 12 to the outermost end portion of the cutting unit 12. In the related art, commonly, a cutting unit is formed to have a cross-sectional area greater than 50% of an imaginary circle having a radius R corresponding to a distance from a center of the cutting unit to an outermost end portion of the cutting unit.

The drill 10 receives driving power from the driving device (not shown) and rotatably collects bone particles from the patient.

The shaft unit 11 extends in a direction and is connected to the driving device. The shaft unit 11 starts to rotate when receiving the driving power from the driving device. Referring to FIG. 1, the driving device is connected to an upper portion of the shaft unit 11, whereas the cutting unit 12 is arranged at a lower portion of the shaft unit 11.

In the present embodiment, the shaft unit 11 and the cutting unit 12 are connected to each other via a connecting unit 13. In other words, the shaft unit 11 is connected to an upper end portion of the connecting unit 13, whereas the cutting unit 12 is connected to a lower end portion of the connecting unit 13.

The cutting unit 12 revolves together with the shaft unit 11, cuts into an autogenous bone of a patient, and collects bone particles from the patient. The cutting unit 12 extends spirally in the direction in which the shaft unit 11 extends.

Referring to FIG. 1, the cutting unit 12 includes a first blade 121 and a second blade 122.

The first and second blades 121 and 122 extend spirally downward from the connecting unit 13, at a same pitch. Flutes that are formed when the first blade 121 and the second blade 122 spirally extend, are provided as the storage space 26 in which bone particles collected from the anautogenous bone of a patient are stored.

As shown in FIG. 2, two or more discontinuously sloped surfaces having different slopes are formed at an end portion of the cutting unit 12. In the present embodiment, the end portions of the cutting unit 12, that is, leading end portions of the first and second blades 121 and 122, are formed to have two-stage discontinuously sloped surfaces.

In detail, the two-stage discontinuously sloped surfaces are denoted by the reference numerals 121a, 121b, 122a, and 122b The discontinuously sloped surfaces 121a and 121b having different slopes are formed at the leading end portions of the first blade 121, whereas the discontinuously sloped surfaces 122a and 122b having different slopes are formed at the leading end portions of the second blade 122. The discontinuously sloped surfaces 121a and 122a have same slope, and the discontinuously sloped surfaces 121b and 122b have same slope. The slope of the discontinuously sloped surfaces 121a and 122a is smaller than that of the discontinuously sloped surfaces 121b and 122b.

FIG. 3 is a cross-sectional view of the cutting unit 12. An outermost thickness W1 of the first blade 121 or the second blade 122 is greater than a thickness W2 of a center portion of the first blade 121 or the second blade 122.

That is, the thickness of the first blade 121 and the second blade 122 decreases toward the center portion of the cutting unit 12. Accordingly, the storage space 26 for housing bone particles collected during revolutions of the first and second blades 121 and 122 may become larger.

Referring to FIG. 1, the end portion of the cutting unit 12 includes a sloped surface 123. Thus, the center portion of the cutting unit 12 has a pointed shape. Furthermore, a point 124 that protrudes and has a greater slope than the sloped surface 123 is attached to the center portion of the end portion of the cutting unit 12. The point 124 is formed as a single body with the cutting unit 12.

When the cutting unit 12 approaches an autogenous bone of a patient, the pointer 124 allows the cutting unit 12 to easily move and contact the autogenous bone.

Furthermore, when the cutting unit 12 is rotating, the pointer 124 reduces shaking of the cutting unit 12. Accordingly, the point 124 allows the cutting unit 12 to precisely approach the autogenous bone for collecting bone particles and reduces a rocking motion of the cutting unit 12 during implant surgery.

According to the present embodiment, the drill 10 further includes a stopper 14 and first and second protrusions 15 and 16.

The stopper 14 protrudes from an outer circumferential surface of the drill 10. In the present embodiment, the stopper 14 is arranged above the connecting unit 13 and protrudes outward. A movement of a drill cover 30 described below is restricted by the stopper 14. In detail, when the drill cover 30 moves upward, the drill cover 30 is stopped from moving by the stopper 14.

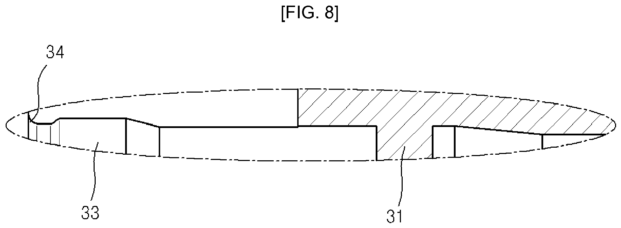

The first protrusion 15 is formed below the stopper 14. In the present embodiment, the first protrusion 15 protrudes along an outer circumferential surface of the connecting unit 13. Hook protrusions 34 (see FIG. 8) of the drill cover 30 described below are stopped by the first protrusion 15.

The second protrusion 16 is formed below the first protrusion 15. In the present embodiment, the second protrusion 16 protrudes along the outer circumferential surface of the connecting unit 13. The second protrusion 16 is also stopped by the hook protrusions 34 of the drill cover 30 described below. In detail, the hook protrusions 34 are stopped by the first protrusion 15 after being stopped by the second protrusion 16. Detailed descriptions thereof will be given below.

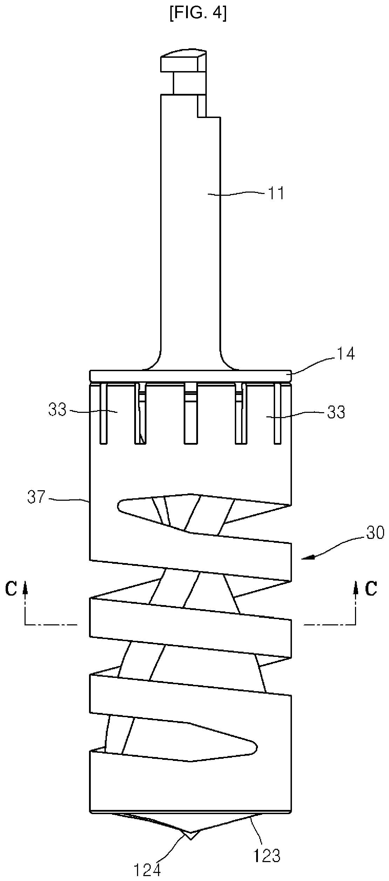

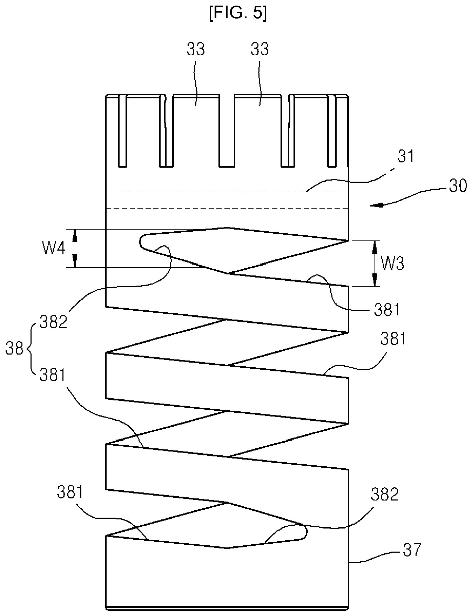

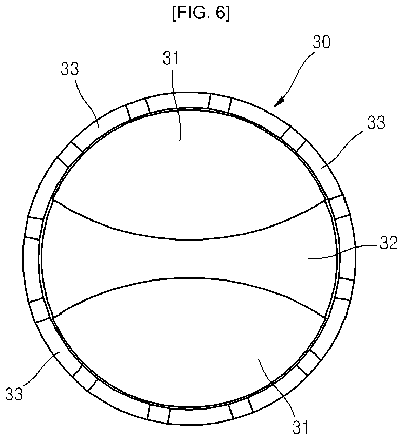

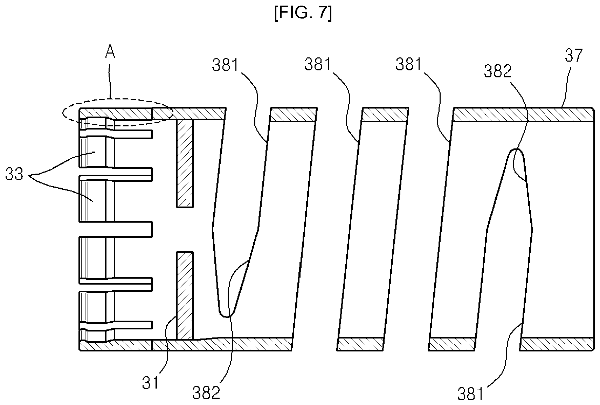

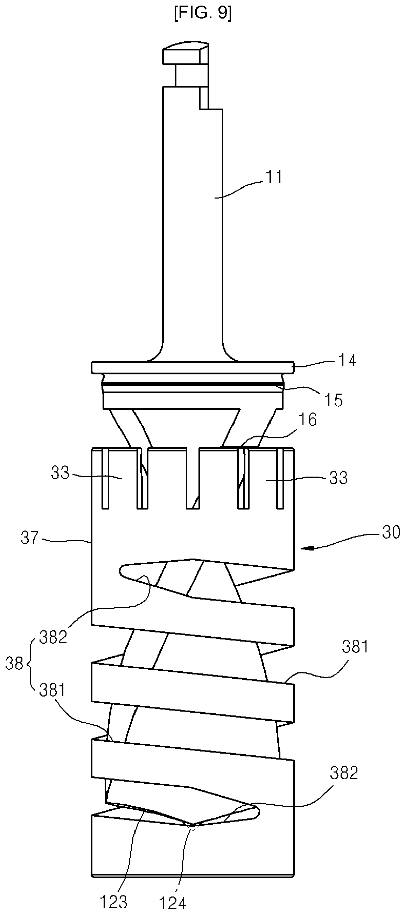

FIG. 4 is a front view of the autogenous bone collector according to an embodiment of the present invention. FIG. 5 is a front view of a drill cover of the autogenous bone collector shown in FIG. 4. FIG. 6 is a plan view of the drill cover shown in FIG. 5. FIG. 7 is a sectional view of the drill cover shown in FIG. 5. FIG. 8 is a diagram showing a section slightly different to section "A" of FIG. 7 in closer detail. FIG. 9 is a diagram showing a primary combination of the drill and the drill cover. FIG. 10 is a sectional view along a line C-C of FIG. 4.

Referring to FIGS. 4 and 5, a drill cover 30 is coupled to the drill 10 having the cutting unit 12 which rotatably collects bone particles from the patient.

The storage space 26 for housing bone particles is formed between an inner circumferential surface 39 of the drill cover 30 and a cutting surface ℓ of the cutting unit 12 (refer to FIG. 10). The drill cover 30 allows bone particles collected by the drill 10 to be collected within the storage space 26 without falling out of the drill cover 30. The drill cover 30 includes a main body 37 and a cut-open portion 38.

A top and a bottom of the main body 37 are open and the main body 37 is coupled to an outer area of the cutting unit 12. The drill 10 is inserted into and combined with the main body 37.

The main body 37 is formed of titanium nitride (TiN) or stainless steel. Accordingly, the main body 37 may be repeatedly used and has high durability. During sterilization of the main body 37, a structure of the main body 37 remains unchanged even at a high temperature. Therefore, the main body 37 may be continuously used.

An end portion of the main body 37 is located close to an end portion of the cutting unit 12. In detail, the end portion of the main body 37 is located slightly above the end portion of the cutting unit 12. In other words, as shown in FIG. 4, when the drill cover 30 is coupled to the drill 10, a part of the end portion of the cutting unit 12 slightly protrudes out of the end portion of the drill cover 30.

By locating the end portion of the main body 37 close to the end portion of the cutting unit 12, bone particles collected at the beginning of the rotation motion of the cutting unit 12 are prevented from falling out of the drill cover 30.

The cut-open portion 38 may be compressed in a direction in which the upper portion and the lower portion of the main body 37 approach each other. The cut-open portion 38 is cut in a spiral shape in the main body 37.

The cut-open portion 38 extends downward in a spiral shape. Therefore, since the cut-open portion 38 is formed by spirally cutting the outer circumferential surface of the main body 37, the drill cover 30 has a spring-like structure.

Referring to FIG. 5, the cut-open portion 38 may be formed to have a constant pitch. Furthermore, although not shown, the cut-open portion 38 may have a varying pitch. A force for compressing the drill cover 30 may be adjusted by changing the pitch of the cut-open portion 38. In other words, a tension of the drill cover 30 may be adjusted.

For example, a force for compressing the drill cover 30 may be reduced by forming the upper portion and the lower portion of the cut-open portion 38 to have a same pitch and forming the middle portion of the cut-open portion 38 to have a pitch shorter than the pitches of the upper portion and the lower portion of the cut-open portion 38. The cut-open portion 38 may be manufactured in variousmanners.

According to the present embodiment, the cut-open portion 38 includes a first cut-open portion 381 and a second cut-open portion 382.

The first cut-open portion 381 has a first cut-open width W3 that is constant. The second cut-open portion 382 has a second cut-open width W4 smaller than the first cut-open width W3.

The second cut-open portion 382 extends from two opposite ends of the first cut-open portion 381, so that a width of the second cut-open portion 382 gradually decreases toward the end portions thereof.

When the drill cover 30 is compressed, the first cut-open width W3 of the first cut-open portion 381 and the second width W4 of the second cut-open portion 382 decrease. The tapered shape of the second cut-open portion 382 induces easy compression at the end portion of the second cut-open portion 382 to prevent collected bone particles from falling out of the drill cover 30.

The drill cover 30 further includes partitioning wall 31 and protruding parts 33.

Referring to FIG. 6, the partitioning wall 31 extends from an inner circumferential surface of the main body 37. The partitioning wall 31 includes a housing portion 32 through which the cutting surface ℓ of the cutting unit 17 may pass.

The partitioning wall 31 includes two walls. The two walls extend in a direction in which the two walls approach each other from the inner circumferential surface of the main body 37. The housing portion 32 in which the cutting unit 12 is housed is formed between the two walls.

The housing portion 32 has a shape corresponding to a shape of the cutting surface ℓ of the cutting unit 12. When the drill cover 30 is coupled to the drill 10, the cutting unit 12 is inserted into the housing portion 32.

The partitioning wall 31 helps collection of bone particles by scraping bone particles attached to the outer circumferential surface of the cutting unit 12 when the drill cover 30 is detached from the cutting unit 12.

Referring to FIGS. 5 and 6, the protruding parts 33 are formed at the upper portion of the drill cover 30. In the present embodiment, the protruding parts 33 protrude upward from the upper end of the drill cover 30 and are spaced apart from each other at a constant interval in a circumferential direction.

The protruding parts 33 are arranged at the upper end of the drill cover 30. However, a number of the protruding parts 33 and an interval therebetween may vary. In another embodiment, the protruding parts 33 may be spaced apart from each other at different intervals.

Referring to FIGS. 7 and 8, the hook protrusions 34 that are bent inward are arranged at the upper ends of protruding parts 23. The hook protrusions 34 are primarily stopped by the second protrusion 16 when the drill cover 30 is coupled to the cutting unit 12 and are secondarily stopped by the first protrusion 15 when the drill cover 30 moves further upward.

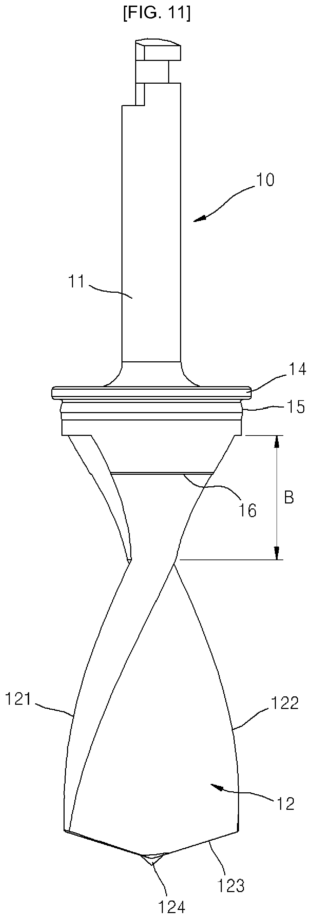

Furthermore, the cutting unit 12 has a constant outermost width. FIG. 11 show amodification example of the drill 10 according to another embodiment. As shown in FIG. 11, the cutting unit 12 may be formed such that an outermost width of the cutting unit 12 increases toward the shaft unit 11 (section B of FIG. 11).

Hereinafter, an operation of the autogenous bone collector including the drill cover 30 will be described in detail.

Referring to FIG. 9, an end portion of the cutting unit 12 is brought close to the upper end of the drill cover 30 (the protruding parts 33) and the cutting unit 12 is coupled to the drill cover 30. Since the cutting unit 12 may pass through the housing portion 32 formed by the partitioning wall 31, the hook protrusions 34 are moved to a location at which the hook protrusions 34 are stopped by the second protrusion 16 by rotating the cutting unit 12 and the drill cover 30 with respect to each other. During this process, the cutting unit 12 is primarily combined with the drill cover 30.

When the shaft unit 11 further rotates, the drill cover 30 moves upward along the flutes formed by the first and second blades 121 and 122.

As shown in FIG. 4, the drill cover 30 moves to a location at which the drill cover 30 is stopped by the stopper 14, and the hook protrusions 34 are stopped by the first protrusion 15. Therefore, relative locations of the cutting unit 12 and the drill cover 30 are set. The protruding parts 33 are slightly elastically-deformed when the hook protrusions 34 are stopped by the first and second protrusions 15 and 16, and thus the drill cover 30 is easily coupled to the drill 10.

The point 124 of the cutting unit 12 is located on an autogenous bone of a patient. Since the point 124 has a greater slope than the sloped surface 123 of the cutting unit 12, the point 124 may be located precisely at a location on the autogenous bone from which bone particles are to be collected.

The driving device is connected to the shaft unit 11 and the shaft unit 11 starts rotating by applying driving power thereto. The cutting unit 12 cuts into the autogenous bone while pushing the drill 10 towards the autogenous bone. At the beginning of the rotation motion of the cutting unit 12, the lower portion of the main body 37 is located close to the autogenous bone. Therefore, bone particles collected when the cutting unit 12 starts rotating are prevented from falling out of the drill cover 30.

When the drill 10 is pushed toward the autogenous bone, the main body 37 of the drill cover 30 is compressed. Accordingly an end portion of the cutting unit 12 gradually protrudes below the main body 37.

The end portion of the cutting unit 12 continues to protrude until the main body 37 is fully compressed. Thus, a length of a portion of the cutting unit 12 which may protrude below the drill cover 30 is limited. Therefore, the length of the portion of the cutting unit 12 penetrating into the autogenous bone may be maintained constant, and thus a depth of the portion of the cutting unit 12 penetrating into the autogenous bone may be controlled. Thus, the length of the portion of the cutting unit 12 protruding below the drill cover 30 may be adjusted by adjusting a length of the main body 37 of the drill cover 30. While the cutting unit 12 is rotating, collected bone particles are housed in the storage space 26.

When collection of bone particles is completed, the drill cover 30 is detached from the cutting unit 12 by rotating it with respect to the cutting unit 12.At this time, the partitioning wall 31 scrapes bone particles accumulated on the cutting unit 12.

Thus, bone particles between the first and second blades 121 and 122 are scraped by the partitioning wall 31 and fall in the drill cover 30. Therefore, the bone particles are automatically removed from the cutting unit 12.

As described above, since the lower end portion of the drill cover 30 may be located close to the autogenous bone, bone particles collected when the cutting unit 12 starts rotating are prevented from falling out of the drill cover 30.

Furthermore, since the drill cover 30 has a spring-like shape, a length of the portion of the cutting unit 12 cutting into the autogenous bone of the patient may be maintained constant, thereby improving stability during surgery.

Furthermore, since the drill cover 30 is formed of TiN or stainless steel, the drill cover 30 has improved durability and may be repeatedly used. Furthermore, the shape of the drill cover 30 may remain unchanged even during high-temperature sterilization.

Furthermore, since the drill cover 30 includes the partitioning wall 31, the bone particles attached to the outer circumferential surface of the cutting unit 12 may be scraped easily. Therefore, a period of time for performing the surgery may be significantly reduced.

Also, the autogenous bone collector according to an embodiment of the present invention may collect a large number of bone particles because the bone particles collected by the cutting unit 12 are housed in a storage space larger than that in the related art.

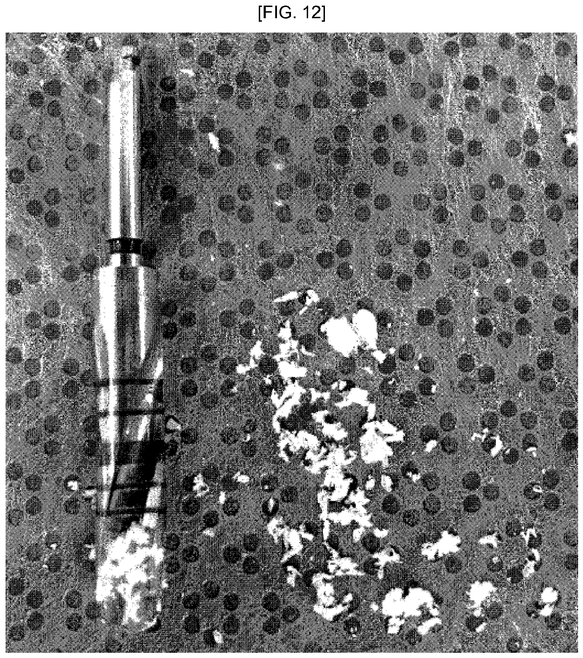

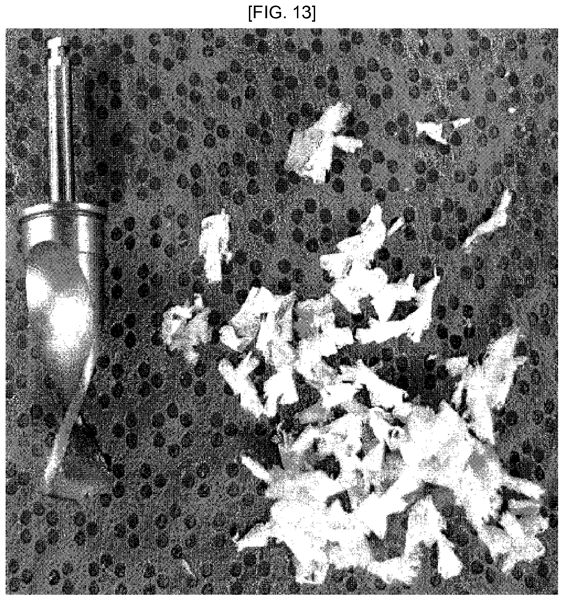

Furthermore, the drill 10 according to the example embodiments of the present disclosure includes two or more discontinuously sloped surfaces having different slopes at leading end portions thereof. Thus, the cutting efficiency of the drill 10 is significantly improved compared to the cutting efficiency of an existing drill. FIGS. 12 and 13 respectively show photographs of bone particles collected by the autogenous bone collector according to an example embodiment of the present invention and bone particles collected by an existing autogenous bone collector.

Referring to FIGS. 12 and 13, sizes of bone particles collected by the autogenous bone collector according to an example embodiment of the present invention are significantly larger than those collected by an existing autogenous bone collector. Therefore, the improved cutting efficiency of the autogenous bone collector according to an example embodiment enables collection of larger bone particles that are better suited for dental implant surgery.

According to the example embodiment of the present invention, the autogenous bone collector including the drill and drill cover collects bone particles during dental implant surgery, and has increased capacity of housing bone particles and is capable of easily separating bone particles from a cutting unit, controlling a depth of the cutting unit that penetrates into an autogenous bone, and housing bone particles collected by the drill within the drill cover while preventing these from falling out of the drill cover.

While the present invention has been particularly shown and described with reference to the example embodiments thereof, it will be understood by those of ordinary skill in the art that various changes in form and details may be made therein without departing from the scope of the present invention as defined by the following claims.

## Claims

1. An autogenous bone collector comprising:
a drill (10) having a shaft unit (11) connectable to a driving device, and a cutting unit (12), which is configured to rotate and to collect bone particles of a patient; and
a drill cover (30), which comprises a main body (37) that has the top and the bottom open and is coupled to the exterior of the cutting unit (12), the cutting unit (12) having a cutting surface (ℓ), the drill cover (30) having an inner circumferential surface (39) and forming a storage space (26) for housing bone particles between the inner circumferential surface (39) and the cutting surface (ℓ),
**characterised in that** the main body (37) is compressible in a direction in which the upper portion and the lower portion of the main body (37) approach each other.

2. The autogenous bone collector of claim 1, wherein a cut-open portion (38), which includes spiral cuts, is formed in the main body (37).

3. The autogenous bone collector of claim 2, wherein the width of the cut-open portion (38) is constant.

4. The autogenous bone collector of claim 2, wherein the cut-open portion (38) comprises:
a first cut-open portion (381) having a constant first cut-open width; and
a second cut-open portion (382) having a second cut-open width smaller than the first cut-open width.

5. The autogenous bone collector of claim 4, wherein the second cut-open portion (382) extends from the two opposite ends of the first cut-open portion (381), and the width of the second cut-open portion (382) gradually decreases toward an end portion thereof.

6. The autogenous bone collector of claim 1, comprising protruding parts (33) protruding upward from the upper end of the drill cover (30) to be spaced apart from each other at a constant interval in the circumferential direction.

7. The autogenous bone collector of claim 1, further comprising a partitioning wall (31), which extends from the inner circumferential surface of the drill cover (30), wherein the partitioning wall (31) comprises a housing portion (32) through which the cutting surface of the cutting unit (12) is configured to pass.

8. The autogenous bone collector of claim 1, wherein hook protrusions (34) that are bent inward are arranged at the upper end of main body (37).

9. The autogenous bone collector of claim 1, wherein the main body (37) is formed of titanium or stainless steel.

10. The autogenous bone collector of claim 1, wherein the cross-sectional area (L) of the cutting unit (12) is from 10% to 40% of an area of an imaginary circle having a radius (R) corresponding to a distance from the center of the cutting unit (12) to the outermost end portion of the cutting unit (12).

11. The autogenous bone collector of claim 1, wherein the cutting unit (12) comprises a stopper (14) protruding from the outer circumferential surface of the drill (10),
movement of the drill cover (30) is restricted by the stopper, and
a first protrusion (15) is formed between the stopper (14) and the end portion of the cutting unit (12).

12. The autogenous bone collector of claim 11, wherein a second protrusion (16) is formed between the first protrusion (15) and the end portion of the cutting unit (12).

13. The autogenous bone collector of claim 1, wherein the cutting unit (12) comprises:
a first blade (121), which spirally extends; and
a second blade (122), which spirally extends at a same pitch as the first blade.

14. The autogenous bone collector of claim 1, wherein the end portion of the cutting unit (12) comprises two or more discontinuously sloped surfaces (121a, 121b, 122a, 122b) having different slopes.

15. The autogenous bone collector of claim 1, wherein the cutting unit (12) comprises a point (124) that protrudes from the center portion of the end portion of the cutting unit (12).

## Patentansprüche

1. Autogener Knochensammler, aufweisend:
einen Bohrer (10) mit einer mit einer Antriebsvorrichtung verbindbaren Schafteinheit (11) und mit einer Schneideinheit (12), die ausgelegt ist zu rotieren und Knochenteilchen eines Patienten zu sammeln; und
einer Bohrerabdeckung (30), welche einen an der Ober- und Unterseite offenen Hauptkörper (37) aufweist und an das Äußere der Schneideinheit (12) gekoppelt ist, wobei die Schneideinheit (12) eine Schneidfläche (t) aufweist, die Bohrerabdeckung (30) eine innere umfängliche Fläche (39) aufweist und einen Speicherraum (26) zur Aufnahme der Knochenteilchen zwischen der inneren umfänglichen Fläche (39) und der Schneidfläche (t) ausbildet,
**dadurch gekennzeichnet, dass** der Hauptkörper (37) in einer Richtung komprimierbar ist, in welcher sich der obere Bereich und der untere Bereich des Hauptkörpers (37) einander annähern.

2. Autogener Knochensammler nach Anspruch 1, bei dem in dem Hauptkörper (37) ein ausgeschnittener Bereich (38) gebildet ist, der spiralförmige Schnitte aufweist.

3. Autogener Knochensammler nach Anspruch 2, bei dem die Breite des ausgeschnittenen Bereichs (38) konstant ist.

4. Autogener Knochensammler nach Anspruch 2, bei dem der ausgeschnittene Bereich (38) aufweist:
einen ersten ausgeschnittenen Bereich (381) mit einer konstanten Breite des ersten Ausschnitts; und
einen zweiten ausgeschnittenen Bereich (382) mit einer zweiten ausgeschnittenen Breite, die geringer ist als die erste ausgeschnittene Breite.

5. Autogener Knochensammler nach Anspruch 4, bei dem sich der zweite ausgeschnittene Bereich (382) von den beiden gegenläufigen Enden des ersten ausgeschnittenen Bereichs (381) erstreckt, und die Breite des zweiten ausgeschnittenen Bereichs (382) graduell in Richtung seines Endbereichs abnimmt.

6. Autogener Knochensammler nach Anspruch 1, aufweisend vorragende Teile (33), die von einem oberen Ende der Bohrerabdeckung (30) in einer voneinander mit konstantem Abstand in der Umfangsrichtung beabstandeten Form vorragen.

7. Autogener Knochensammler nach Anspruch 1, weiter aufweisend eine Trennwand (31), die sich von der inneren umfänglichen Fläche der Bohrerabdeckung (30) erstreckt, wobei die Trennwand (31) einen Aufnahmebereich (32) so gestaltet aufweist, dass die Schneidfläche der Schneideinheit (12) dadurch hindurchgelangt.

8. Autogener Knochensammler nach Anspruch 1, bei dem Hakenvorsprünge (34), die nach innen gebogen sind, an dem oberen Ende des Hauptkörpers (37) angeordnet sind.

9. Autogener Knochensammler nach Anspruch 1, bei dem der Hauptkörper (37) aus Titan oder rostfreiem Stahl gebildet ist.

10. Autogener Knochensammler nach Anspruch 1, bei dem die Querschnittsfläche (L) der Schneideinheit (12) von 10% bis 40% eines Bereichs eines imaginären Kreises mit Radius R ausmacht, der einem Abstand von der Mitte der Schneideinheit (12) zum äußersten Endbereich der Schneideinheit (12) entspricht.

11. Autogener Knochensammler nach Anspruch 1, bei dem die Schneideinheit (12) einen von der äußeren umfänglichen Fläche des Bohrers (10) vorragenden Stopper (14) aufweist, die Bewegung der Bohrerabdeckung (30) durch den Stopper begrenzt ist, und ein erster Vorsprung (15) zwischen dem Stopper (14) und dem Endbereich der Schneideinheit (12) gebildet ist.

12. Autogener Knochensammler nach Anspruch 11, bei dem ein zweiter Vorsprung (16) zwischen dem ersten Vorsprung (15) und dem Endbereich der Schneideinheit (12) gebildet ist.

13. Autogener Knochensammler nach Anspruch 1, bei dem die Schneideinheit (12) aufweist:
eine erste Klinge (121), die sich spiralförmig erstreckt; und
eine zweite Klinge (122), welche sich mit der gleichen Ganghöhe wie die erste Klinge spiralförmig erstreckt.

14. Autogener Knochensammler nach Anspruch 1, bei dem der Endbereich der Schneideinheit (12) zwei oder mehrere diskontinuierlich geneigte Flächen (121a, 121b, 122a, 122b) aufweist, die unterschiedliche Neigungen haben.

15. Autogener Knochensammler nach Anspruch 1, bei dem die Schneideinheit (12) eine von dem zentralen Bereich des Endbereichs der Schneideinheit (12) vorragende Spitze (124) aufweist.

## Revendications

1. Dispositif collecteur d'autogreffe osseuse comprenant :
un foret (10) ayant une unité de tige (11) pouvant être reliée à un dispositif d'entraînement, et une unité de coupe (12) qui est configurée pour tourner et pour collecter des particules d'os d'un patient ; et
un recouvrement de foret (30) qui comprend un corps principal (37) dont le haut et le bas sont ouverts et qui est couplé à l'extérieur de l'unité de coupe (12), l'unité de coupe (12) ayant une surface de coupe (ℓ), le recouvrement de foret (30) ayant une surface circonférentielle intérieure (39) et formant un espace de stockage (26) pour loger des particules d'os entre la surface circonférentielle intérieure (39) et la surface de coupe (ℓ),
**caractérisé en ce que** le corps principal (37) peut être comprimé dans une direction dans laquelle la portion supérieure et la portion inférieure du corps principal (37) s'approchent l'une de l'autre.

2. Dispositif collecteur d'autogreffe osseuse selon la revendication 1, dans lequel une portion à découpe ouverte (38), qui inclut des découpes en spirales, est formée dans le corps principal (37).

3. Dispositif collecteur d'autogreffe osseuse selon la revendication 2, dans lequel la largeur de la portion à découpe ouverte (38) est constante.

4. Dispositif collecteur d'autogreffe osseuse selon la revendication 2, dans lequel la portion à découpe ouverte (38) comprend :
une première portion à découpe ouverte (381) ayant une première largeur de découpe ouverte constante ; et
une deuxième portion à découpe ouverte (382) ayant une deuxième largeur de découpe ouverte plus petite que la première largeur de découpe ouverte.

5. Dispositif collecteur d'autogreffe osseuse selon la revendication 4, dans lequel la deuxième portion à découpe ouverte (382) s'étend depuis les deux extrémités opposées de la première portion à découpe ouverte (381), et la largeur de la deuxième portion à découpe ouverte (382) diminue progressivement vers une portion d'extrémité de celle-ci.

6. Dispositif collecteur d'autogreffe osseuse selon la revendication 1, comprenant des parties proéminentes (33) dépassant vers le haut depuis l'extrémité supérieure du recouvrement de foret (30) qui sont espacées les unes des autres à un intervalle constant dans la direction circonférentielle.

7. Dispositif collecteur d'autogreffe osseuse selon la revendication 1, comprenant en outre une paroi de séparation (31) qui s'étend depuis la surface circonférentielle intérieure du recouvrement de foret (30),
dans lequel la paroi de séparation (31) comprend une portion de logement (32) au travers de laquelle la surface de coupe de l'unité de coupe (12) est configurée pour passer.

8. Dispositif collecteur d'autogreffe osseuse selon la revendication 1, dans lequel des proéminences en forme de crochet (34), qui sont courbées vers l'intérieur, sont disposées à l'extrémité supérieure du corps principal (37).

9. Dispositif collecteur d'autogreffe osseuse selon la revendication 1, dans lequel le corps principal (37) est formé de titane ou d'acier inoxydable.

10. Dispositif collecteur d'autogreffe osseuse selon la revendication 1, dans lequel la zone de section transversale (L) de l'unité de coupe (12) est de 10 % à 40 % d'une zone d'un cercle imaginaire ayant un rayon (R) correspondant à une distance du centre de l'unité de coupe (12) à la portion d'extrémité la plus éloignée de l'unité de coupe (12).

11. Dispositif collecteur d'autogreffe osseuse selon la revendication 1, dans lequel l'unité de coupe (12) comprend un arrêt (14) dépassant de la surface circonférentielle extérieure du foret (10),
un mouvement du recouvrement de foret (30) étant limité par l'arrêt, et
une première proéminence (15) étant formée entre l'arrêt (14) et la portion d'extrémité de l'unité de coupe (12).

12. Dispositif collecteur d'autogreffe osseuse selon la revendication 11, dans lequel une deuxième proéminence (16) est formée entre la première proéminence (15) et la portion d'extrémité de l'unité de coupe (12).

13. Dispositif collecteur d'autogreffe osseuse selon la revendication 1, dans lequel l'unité de coupe (12) comprend :
une première lame (121) qui s'étend en spirale ; et
une deuxième lame (122) qui s'étend en spirale selon un même pas que la première lame.

14. Dispositif collecteur d'autogreffe osseuse selon la revendication 1, dans lequel la partie d'extrémité de l'unité de coupe (12) comprend deux ou plusieurs surfaces inclinées de façon discontinue (121a, 121b, 122a, 122b) ayant des pentes différentes.

15. Dispositif collecteur d'autogreffe osseuse selon la revendication 1, dans lequel l'unité de coupe (12) comprend un point (124) qui dépasse de la portion centrale de la portion d'extrémité de l'unité de coupe (12).
